(19) **Europäisches Patentamt / European Patent Office / Office européen des brevets**

(11) **EP 1 818 066 A1**

(12) **EUROPEAN PATENT APPLICATION**
published in accordance with Art. 158(3) EPC

(43) Date of publication:
**15.08.2007 Bulletin 2007/33**

(51) Int Cl.:
**A61L 31/00** *(2006.01)*

(21) Application number: **05800319.5**

(22) Date of filing: **01.11.2005**

(86) International application number:
**PCT/JP2005/020099**

(87) International publication number:
**WO 2006/049160 (11.05.2006 Gazette 2006/19)**

(84) Designated Contracting States:
**AT BE BG CH CY CZ DE DK EE ES FI FR GB GR HU IE IS IT LI LT LU LV MC NL PL PT RO SE SI SK TR**

(30) Priority: **05.11.2004 JP 2004322019**

(71) Applicant: **Kaneka Corporation**
**Kita-ku**
**Osaka-shi, Osaka 530-8288 (JP)**

(72) Inventors:
• **USHIZAKI, Koshin,**
**c/o KANEKA CORPORATION**
**Settsu-shi,**
**Osaka 566-0072 (JP)**

• **NAKATANI, Masaru,**
**c/o KANEKA CORPORATION**
**Settsu-shi,**
**Osaka 566-0072 (JP)**
• **KOBAYASHI, Akira,**
**c/o KANEKA CORPORATION**
**Settsu-shi,**
**Osaka 566-0072 (JP)**
• **NISHIMOTO, Takehiro,**
**c/o KANEKA CORPORATION**
**Settsu-shi,**
**Osaka 566-0072 (JP)**

(74) Representative: **HOFFMANN EITLE**
**Patent- und Rechtsanwälte**
**Arabellastrasse 4**
**81925 München (DE)**

(54) **METHOD OF MODIFYING BLOOD CONTACT MATERIAL AND BLOOD CONTACT MATERIAL INHIBITED FROM ACTIVATING GRANULOCYTE**

(57)    An object of the present invention is to provide a method of modifying a blood contact material capable of reducing granulocyte activating action of the blood contact material in medical equipment used for parts which get contact with the blood. The present invention provides a method of modifying blood contact material **characterized by** granulocyte activating action when in contact with blood, the modifying blood contact material is considerably reduced compared with an original blood contact material by immobilizing a tryptophan derivative and a polyanionic compound to the blood contact material. Examples of the tryptophan derivative include tryptophan. Further, examples of the polyanionic compound include dextran sulfate.

EP 1 818 066 A1

**Description**

FIELD OF THE INVENTION

The present invention relates to a method of modifying a surface of a material used in medical field in the parts which get contact with blood, the method inhibiting a granulocyte activating action of the material, and relates to a blood contact material inhibited from granulocyte activation.

BACKGROUND ART

The adhesiveness of granulocyte increases upon recognition of a foreign substance, and the granulocyte releases reactive oxygen together with various enzymes contained in an azurophilic granule. Granulocyte elastase and myeloperoxidase are eutral proteases stored in the azurophilic granule, and the concentration thereof is measured generally as an indicator of granulocyte activation. From a previous study, it is presumed that the rise in concentration of the granulocyte elastase and myeloperoxidase is caused by a mechanism other than complement activation (Nonpatent literature 1).

As the clinical effects of granulocyte activation, there have been pointed out the possibilities that the reactive oxygen causes a vascular disorder such as arteriosclerosis via oxidation of lipid like LDL (oxidized LDL) (Nonpatent literature 2) and that weakening of an erythrocyte membrane by peroxidation of erythrocyte membrane lipid is one of the causes of anemia (Nonpatent literature 3).

In recent years, in medical care where an artifact and blood get contact with each other, typified by stent placement into a blood vessel in hemodialysis in the field of blood purification and in the field of cardiovascular surgery, recognition of a foreign substance to a contact material by granulocyte which occupies a large part of leukocyte has become a problem.

Therefore, the development of a method of modifying a blood contact material capable of reducing the granulocyte activating action of the blood contact material has been desired.

Nonpatent literature 1: J.Boehler et.al.Nephrology Dialysis Transplantation, vol.8, p.1359-1365, 1993
Nonpatent literature 2: Higuchi et.al., The Japanese Journal of Artificial Organs, vol.26 No. 4: p.835-839, 1997
Nonpatent literature 3: S.S.Schmidtmann et.al., Nephrology Dialysis Transplantation, vol.5, p.600-603, 1990

DISCLOSURE OF THE INVENTION

PROBLEMS TO BE SOLVED BY THE INVENTION

An object of the present invention is to provide a method of modifying a blood contact material which can reduce granulocyte activating action of the blood contact material; the blood contact material modified by the method; a blood contact circuit, an indwelling device, and a blood purification system using the modified blood contact material.

MEANS FOR SOLVING THE PROBLEMS

The present inventors extensively examined the methods of modifying a blood contact material capable of inhibiting granulocyte activating action of the blood contact material. As the result, it was found that granulocyte activating action when in contact with blood is considerably reduced compared with an original blood contact material by immobilizing, to the blood contact material, a polyanionic compound (s) in an amount of 6. 4 nmol or more and 96.2 nmol or less per $1m^2$ of the surface area of the material and a tryptophan derivative (s) in a molar ratio to the immobilization amount of the polyanionic compound (s) of 1 or more and 70 or less, and accomplished the present invention.

Namely, the present invention relates to a method of modifying the blood contact material wherein a polyanionic compound (s) is immobilized in an amount of 6.4 nmol or more and 96.2 nmol or less per $1m^2$ of the surface area, and at the same time a tryptophan derivative(s) is immobilized with a molar ratio to the immobilization amount of the polyanionic compound (s) of 1 or more and 70 or less, wherein the granulocyte activating action when brought into contact with blood is considerably reduced compared with the original blood contact material by the modification.

The present invention further relates to the blood contact material modified by the method.

The present invention further relates to a blood contact circuit, an indwelling device, and a blood purification system using the modified blood contact material in parts which are in contact with blood.

EFFECTS OF THE INVENTION

By the method of modifying the blood contact material of the present invention, granulocyte activating action of the blood contact material can be dramatically reduced so that safer medical equipment can be produced.

BEST MODE FOR CARRYING-OUT OF THE INVENTION

The method of modifying the blood contact material of the present invention comprises immobilizing a polyanionic compound(s) in an amount of 6.4 nmol or more and 96.2 nmol or less per 1 $m^2$ of a surface area of the material and immobilizing a tryptophan derivative(s) with a molar ratio to the immobilization amount of polyanionic compound(s) of 1 or more and 70 or less.

The polyanionic compound of the present invention refers to a compound having a plurality of anionic functional groups in a molecule. The anionic functional group of the present invention refers to a functional group with neutral pH which is charged negatively such as a carboxyl group, a sulfonic acid group, a sulfate ester group and a phosphoric acid ester group.

Typical examples of the polyanionic compound include a synthetic polyanionic compound such as polyacrylic acid, polyvinylsulfonic acid, polystyrene sulfonic acid, polyglutamic acid, polyaspartic acid, polymethacrylic acid, polyphosphoric acid, and a styrene-maleic acid copolymer; a synthetic acid polysaccharide such as dextran sulfate and carboxymethylcellulose; an in vivo-derived acid mucopolysaccharide having a sulfate ester group such as chondroitin sulfate, dermatan sulfate, and keratan sulfate; an acid mucopolysaccharide having a N-sulfonic acid group and a sulfate ester group such as heparin and heparan sulfate; a polysaccharide having an in vivo-derived anionic functional group such as chondroitin phosphomannan; and an in vivo-derived nucleic acid such as deoxyribo nucleic acid and ribonucleic acid, but the present invention is not limited to these examples.

Among the compounds typified by these, it is more practical to use a synthetic compound than to use an in vivo-derived compound (s) without modification for reasons that a highly pure substance can be obtained for low cost and, further, that the amount of anionic functional groups to be introduced can be controlled, and the like. From these points, a synthesized polyanionic compound such as polyacrylic acid, polyvinylsulfuric acid, polyvinylsulfonic acid, polystyrenesulfonic acid, polyglutamic acid, polyaspartic acid, polymethacrylic acid, polyphosphoric acid, and a styrene-maleic acid copolymer; a synthetic acid polysaccharide such as dextran sulfate and carboxy methylcellulose and the like are preferably used. Among them, polyacrylic acid, polystyrene sulphonic acid and dextran sulfate are preferred due to low cost, and in view of safety, dextran sulfate is particularly preferable. The upper limit of the molecular weight of the polyanionic compound is not particularly limited, but it is preferably one million or less from a practical point of view.

In the present invention, there are various methods of immobilizing the polyanionic compound to the blood contact material, and any method may be used. Typical methods include:

> (1) a method in which the polyanionic compound is bonded covalently to the surface by grafting method using radiation or electron beam; and
> (2) a method in which the polyanionic compound is bonded covalently via a functional group by a chemical method. These immobilization methods may be used regardless of the shape of the material.

Among them, considering that the blood contact material of the present invention is a material obtained by immobilizing the polyanionic compound and the tryptophan derivative, it is an easier and thus a preferred method in the present invention since a method in which the polyanionic compound is covalently bonded via a functional group by a chemical method can be performed in a same manner as the immobilization of the tryptophan derivative.

The tryptophan derivative(s) in the present invention refer to tryptophan esters such as tryptophan, tryptophan ethyl ester, tryptophan methyl ester, and compounds having a structure similar to tryptophan having an indole ring(s) such as tryptamine and tryptophanol. These tryptophan derivatives may be any of L-tryptophan, D-tryptophan, DL-tryptophan or a mixture of two or more kinds of the tryptophan derivatives. Among these tryptophan derivatives, tryptophan is preferable in view of safety, and L-tryptophan is preferably used in practice since it is a natural amino acid, and a plenty of safety data thereof is available, and it is easily available at low cost.

As the method of immobilizing the tryptophan derivative in the present invention, a method in which the tryptophan derivative is bonded covalently via a functional group of the blood contact material in chemical method is preferably used.

The immobilization amount of the polyanionic compound of the present invention needs to be 6.4 nmol or more and 96.2 nmol or less per $1m^2$ of the surface area, and the molar ratio of the immobilization amount of the tryptophan derivative to the immobilization amount of the polyanionic compound needs to be 1 or more and 70 or less.

The molar ratio (TR/PA ratio) of the immobilization amount of the tryptophan derivative to the immobilization amount of the polyanionic compound in the present invention is the value calculated by the following formula.

$$\text{TR/PA ratio} = \text{number of moles of immobilized tryptophan derivative per } 1m^2 \text{ of the material surface area/number of moles of immobilized polyanion per } 1m^2 \text{ of the material surface area}$$

The present inventors extensively examined the immobilization amount of the polyanionic compound as well as the immobilization amount of the tryptophan derivative and the degree of granulocyte activation when in contact with blood. As the result, it was found that, surprisingly, making the immobilization amount of the polyanionic compound 6.4 nmol or more and 96.2 nmol or less per $1m^2$ of the surface area and keeping the TR/PA ratio 1 or more and 70 or less provide the effect of reducing granulocyte activation.

In the present invention, the immobilization amount of polyanionic compound is 6.4 nmol or more and 96.2 nmol or less per $1m^2$ of the surface area. When the immobilization amount is less than 6. 4 nmol, the adhesiveness of leukocytes and platelets increases by contact with the material, and the number of leukocytes and platelets in blood decreases. Further, when it is more than 96.2 nmol, the adhesion of substances in blood becomes conspicuous due to negative charge of polyanion. From these two points, it is preferably 7.7 nmol or more and 66.1 nmol or less and most preferably 9. 6 nmol or more and 32.1 nmol or less.

Further, in the present invention, the molar ratio (TR/PA ratio) of the immobilization amount of the tryptophan derivative to the immobilization amount of the polyanionic compound is 1 or more and 70 or less. If the TR/PA ratio departs from the range, the adhesiveness of platelets increases. From this point, it is preferably 5 or more and 60 or less, and most preferably 10 or more and 50 or less.

The methods of measuring the surface area of the blood contact material in the present invention include a method in which it is calculated mathematically by integration, and a method utilizing the determination of the amount of gas adsorption onto the material surface represented by BET method; the latter method is more convenient when measuring a porous surface area. When measuring the surface area by determination of the amount of gas adsorption, the sample needs to be subjected to dry treatment, and in such an occasion, a treatment method which does not destroy the surface of the material such as lyophilization is preferably used.

The methods of measuring the immobilization amount of the polyanionic compound in the present invention include a method in which the content of the atoms contained in the polyanionic compound in the blood contact material is measured (for example, in the case of dextran sulfate, the content of sulfur in the blood contact material is measured), a method in which a dye solution having a property of bonding to the polyanionic compound is brought into contact with the blood contact material, thereby determining the immobilization amount from the decrease in the amount of pigment in the solution. Among them, by the method using the dye solution, the immobilization amount of the polyanionic compound can be measured easily and, further, accurately. More specifically, as the method is shown in Example 1, when the polyanionic compound is dextran sulfate, polyacrylic acid, or the like, utilizing the property of these compounds that it bond to toluidine blue, the immobilization amount can be measured very easily from the amount of adsorption of toluidine blue when the toluidine blue solution is in contact with the blood contact material.

The immobilization amount of the tryptophan derivative of the present invention can be measured utilizing the property that it gets colored when condensing an aldehyde such as p-dimethylbenzaldehyde with a indole ring which is present in a molecule of the tryptophan derivative under highly acidic condition (edited by Koichi Yamada, Aminosan Hakko (Amino Acid Fermentation)" (vol.2) Kakuron (particular subjects), p. 43-45, KYORITSU SHUPPAN CO., LTD, 1972). Alternatively, it can be measured by a method utilizing the property that the indole ring which is present in a molecule of the tryptophan derivative yield a fluorescence having the maximum at the proximity of 350 nm when excited with light in the proximity of 280 nm, or when the material itself is a compound which does not contain nitrogen, it can be measured by measuring the content of nitrogen in the blood contact material as the specific method is shown in Example 1.

The method of modifying the blood contact material of the present invention is applicable without any specific limitation as long as it is medical equipment which contacts with blood. Examples of such medical equipment include a blood contact circuit, an indwelling device, a blood storage container, endovascular equipment and a blood purification system.

The blood contact material of the present invention is solid at normal temperature and pressure, and is water-insoluble. The blood contact material may take any of the shapes of plate, sphere, granule, flat membrane, fiber, or hollow fiber to be used effectively, and is not particularly limited.

The blood contact material of the present invention, regardless of the shape, preferably has a functional group (s) which may be used for bonding in order to immobilize the polyanionic compound and the tryptophan derivative. Typical examples of the functional group include an amino group, an amide group, a carboxyl group, an acid anhydride group, a succinimide group, a hydroxyl group, a thiol group, an aldehyde group, a halogen group, an epoxy group, a silanol group, and a tresyl group, but is not limited to these. Further, these functional groups may have been activated by, for example, halogenation-cyanidation method, epichlorohydrin method, bis-epoxide method, bromoacetyl bromide method and the like. Among them, epichlorohydrin method is in particular preferably used in view of practice and safety.

The materials of the blood contact material of the present invention is not particularly limited, but typical examples include organic materials comprising a polysaccharide such as cellulose, acetic acid cellulose and dextrin, a synthetic polymer such as polystyrene, styrene-divinylbenzene copolymer, polyacrylamide, polyacrylic acid, polymethacrylic acid, polyacrylic ester, polymethacrylic acid ester and polyvinyl alcohol. These may contain a coating layer including a polymer material having a hydroxyl group such as hydroxyethyl methacrylate, or a graft copolymer and the like such as a copolymer of a monomer having a polyethylene oxide chain and another polymerizable monomer. Among these, a synthetic polymer comprising cellulose, polyvinyl alcohol and the like is preferably used in practice since it is easy to introduce an active group on the surface of the material.

The granulocyte elastase in the present invention is occasionally called neutrophil elastase, and they refer to a same substance.

EXAMPLES

The following Examples provide detailed illustration of the method of modifying the blood contact material of the present invention; however, the scope of the present invention is not limited to the Examples.

(Example 1)

Into 100 mL of cellulose beads (sedimentation volume in wet condition) with an average particle diameter of around 450 $\mu$m, 22 ml of water, 34 ml of a 4 N NaOH aqueous solution and 33 ml of epichlorohydrin were added to stir at the temperature of 40 °C for 2 hours to allow the mixture to react. After the reaction, the beads are sufficiently washed with water to obtain epoxidized cellulose beads.

A dextran sulfate aqueous solution containing 9.3 g of dextran sulfate (content of sulfur: approximately 18 %, molecular weight: around 4000) dissolved in 36 ml of water was prepared, and 780 m$^2$ (sedimentation volume of 50 mL), in terms of the surface area, of the epoxidized cellulose beads which were wet with water were added, and the mixture was rendered alkaline with a NaOH aqueous solution before it was reacted at the temperature of 45 °C for 7 hours. After the reaction, the beads were sufficiently washed with water and saline, and subsequently a liquid containing 0.78 g of L-tryptophan dissolved in 40 ml of a diluted NaOH solution was added to react at the temperature of 50 °C for 8 hours. Subsequently, the beads were sufficiently washed with water and saline to obtain modified cellulose beads. The modified cellulose beads thus obtained were charged in the amount of 0.5 ml measured as a sedimentation volume in a polypropylene tube having the inside diameter of 10 mm and a length of 150 mm, and after being wetted with physiological saline, blood of a healthy individual in the amount of 3 ml was added thereto; then the mixture was subjected to batch contact at the temperature of 37 °C for 30 minutes. The concentration of granulocyte elastase before and after contact with the modified cellulose beads was measured by granulocyte elastase measuring kit (Merck Ltd.). Further, as the control, physiological saline alone instead of beads was added in the amount of 0.5 mL to measure the concentration of granulocyte elastase 30 minutes thereafter. As the result, the concentration of granulocyte elastase before contact with the cellulose beads was 55$\mu$g/L, while the concentration of granulocyte elastase after the contact was 90($\mu$g/L). The concentration of granulocyte elastase after the contact with physiological saline alone was 82 ($\mu$g/L)(Table 1).

The surface area of the modified cellulose beads was measured by BET-multipoint method. More specifically, after the modified cellulose beads were dehydrated with ethanol and were subjected to lyophilization treatment in t-butanol, the area per unit weight (specific surface area) was measured by ASAP-2400 (produced by MICROMERITICS). Further, the immobilization amount of tryptophan was obtained from the amount of nitrogen contained in the modified cellulose beads. More specifically, after 1 ml of the modified cellulose beads were sufficiently washed with water and were subjected to drying at the temperature of 60 °C for 6 hours under reduced pressure condition, the immobilization amount of the tryptophan was measured by total nitrogen analyzer. As the result, the immobilization amount of tryptophan in the modified cellulose beads was 435.0 nmol/m$^2$.

The immobilization amount of dextran sulfate was measured by taking advantage of the fact that dextran sulfate and toluidine blue have an affinity for each other. More specifically, into 3 ml of the modified cellulose beads, about 100ml of an aqueous solution of toluidine blue (Basic Blue 17 (Tokyo Chemical Industry Co., Ltd.)) adjusted to approximately 90mg/l was added and stirred for 10 minutes and was left still, and then the toluidine blue which was supernatant was measured by the absorption of light at 630 nm, thereby obtaining the immobilization amount from the amount of decrease. As the result, the immobilization amount of dextran sulfate was 8.1 nmol/m$^2$ and TR/PA ratio was 56.6.

Herein, the volume of the cellulose beads in wet condition is obtained as follows. Namely, the cellulose beads were taken in a measuring container such as a measuring cylinder as slurry immersed in water, and the cellulose beads in the form of slurry in the measuring container is allowed to settle out naturally. After that, a rubber mat is placed in order to keep the measuring container from breaking, and the measuring container is lightly slammed thereto about 5 or 10 times from the height of about 5 to 10 cm in a vertical direction (to the extent that the cellulose beads which have settled out do not soar above excessively) to give vibration. After it is left still for 15 minutes or more, the sedimentation volume of the cellulose beads is read. The operation of vibrating and leaving still is repeated; when the sedimentation volume of the cellulose beads reaches the stage where it no longer changes, it is determined as the sedimentation volume of the cellulose beads under wet condition.

(Comparative Example 1)

The cellulose beads in Example 1 before modification were charged into the polypropylene tube used in Example 1 in the amount of 0.5 ml and were allowed to swell in physiological saline. Blood of the healthy person same as in Example 1 was added thereto in the amount of 3 ml, and was subjected to batch contact at the temperature of 37 °C for 30 min, and the concentration of granulocyte elastase before and after the batch contact was measured. As the result, the concentration of granulocyte elastase after the contact became 134 ($\mu$g/L).

Table 1

| | Concentration of granulocyte elastase | | | |
|---|---|---|---|---|
| | Before contact ($\mu$g/L) | Control ($\mu$g/L) | After contact ($\mu$g/L) | Rate of increase (%) |
| Example 1 | 55 | 82 | 90 | 9.8 |
| Comparative Example 1 | 55 | 82 | 134 | 63.4 |
| Control: Saline alone instead of beads was added. Rate of increase = (After contact - control)/(control)x 100 | | | | |

The results of Table 1 shows that in the cellulose beads of Comparative Example 1, the concentration of granulocyte elastase which is an indicator of granulocyte activation considerably increased after the contact with blood compared with that before contact. Namely, in Comparative Example 1, after the contact of the cellulose beads, the concentration of granulocyte elastase was increased, and taking into account the increase in concentration of granulocyte elastase when physiological saline alone (control) was added, the rate of increase was 63.4 %. On the other hand, in the case of the modified cellulose beads immobilized dextran sulfate and tryptophan in Example 1, the rate of increase was 9.8 %, where the increase in concentration of granulocyte elastase was considerably lowered, showing that the granulocyte activation was inhibited.

**Claims**

1. A method of modifying a blood contact material, **characterized by** inhibiting granulocyte activation by immobilizing a polyanionic compound and a tryptophan derivative to the blood contact material.

2. A method of modifying a blood contact material, wherein immobilization amount of a polyanionic compound per $1m^2$ of surface area is 6.4 nmol or more and 96.2 nmol or less, and molar ratio of immobilization amount of a tryptophan derivative to immobilization amount of the polyanionic compound is 1 or more and 70 or less.

3. The method of modifying the blood contact material according to claim 1 or 2, wherein the polyanionic compound is dextran sulfate.

4. The method of modifying the blood contact material according to any one of claims 1 to 3, wherein the tryptophan derivative is tryptophan.

5. The method of modifying the blood contact material according to any one of claims 1 to 4, wherein the blood contact material comprises a polymeric compound having a hydroxyl group.

6. A blood contact material inhibited from granulocyte activation obtained by immobilizing a polyanionic compound and a tryptophan derivative, wherein immobilization amount of the polyanionic compound is 6.4 nmol or more and 96.2 nmol or less per $1m^2$ of surface area and, at the same time, molar ratio of immobilization amount of the tryptophan derivative to immobilization amount of the polyanionic compound is 1 or more and 70 or less.

7. The blood contact material according to claim 6, wherein the polyanionic compound is dextran sulfate.

8. The blood contact material according to claim 6 or 7, wherein the tryptophan derivative is tryptophan.

9. The blood contact material according to any one of claims 6 to 8, the blood contact material comprising a polymeric compound having a hydroxyl group.

10. A blood contact circuit, using the blood contact material according to any one of claims 6 to 9.

11. A indwelling device, using the blood contact material according to any one of claims 6 to 9.

12. A blood purification system, using the blood contact material according to any one of claims 6 to 9.

## INTERNATIONAL SEARCH REPORT

| | International application No. |
|---|---|
| | PCT/JP2005/020099 |

A. CLASSIFICATION OF SUBJECT MATTER
*A61L31/00*(2006.01), *A61F2/84*(2006.01), *A61M1/14*(2006.01), *A61M1/16*
(2006.01), *A61M1/36*(2006.01)

According to International Patent Classification (IPC) or to both national classification and IPC

B. FIELDS SEARCHED

Minimum documentation searched (classification system followed by classification symbols)
A61F2/84, A61L31/00, A61M1/14, A61M1/16, A61M1/36

Documentation searched other than minimum documentation to the extent that such documents are included in the fields searched
| Jitsuyo Shinan Koho | 1922-1996 | Jitsuyo Shinan Toroku Koho | 1996-2006 |
| Kokai Jitsuyo Shinan Koho | 1971-2006 | Toroku Jitsuyo Shinan Koho | 1994-2006 |

Electronic data base consulted during the international search (name of data base and, where practicable, search terms used)
JSTPLUS(JOIS)

C. DOCUMENTS CONSIDERED TO BE RELEVANT

| Category* | Citation of document, with indication, where appropriate, of the relevant passages | Relevant to claim No. |
|---|---|---|
| A | Miki KANAI et al., "Kessho Kokan Ryoho ni Okeru Ketsueki Nendo Oyobi Kessho Nendo no Henka -Kakushu Chiryoho ni yoru Soi ni Tsuite- (Changes in blood and plasma viscosity during plasmapheresis- Effects of therapeutic method-)", The Japanese Journal of Artificial Organs, 1992, Vol.21, No.3, pages 1137 to 1141 | 1-12 |
| A | JP 57-122875 A (Asahi Chemical Industry Co., Ltd.), 30 July, 1982 (30.07.82), Full text & EP 56977 A1 & US 4432871 A | 1-12 |

| ☒ Further documents are listed in the continuation of Box C. | ☐ See patent family annex. |
|---|---|

| * | Special categories of cited documents: | "T" | later document published after the international filing date or priority date and not in conflict with the application but cited to understand the principle or theory underlying the invention |
|---|---|---|---|
| "A" | document defining the general state of the art which is not considered to be of particular relevance | | |
| "E" | earlier application or patent but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
|---|---|
| 12 January, 2006 (12.01.06) | 24 January, 2006 (24.01.06) |

| Name and mailing address of the ISA/ | Authorized officer |
|---|---|
| Japanese Patent Office | |
| Facsimile No. | Telephone No. |

Form PCT/ISA/210 (second sheet) (April 2005)

8

**INTERNATIONAL SEARCH REPORT**

| International application No. |
|---|
| PCT/JP2005/020099 |

C (Continuation).   DOCUMENTS CONSIDERED TO BE RELEVANT

| Category* | Citation of document, with indication, where appropriate, of the relevant passages | Relevant to claim No. |
|---|---|---|
| A | JP 8-510166 A  (Norusk Hidoro AS.),<br>29 October, 1996 (29.10.96),<br>Full text<br>& WO 1994/026399 A1      & NO 9301809 A<br>& AU 9468169 A            & EP 699103 A1<br>& US 5840190 A | 1-12 |
| A | JP 61-187866 A  (Riken Vitamin Co., Ltd.),<br>21 August, 1986 (21.08.86),<br>Full text<br>(Family: none) | 1-12 |

Form PCT/ISA/210 (continuation of second sheet) (April 2005)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Non-patent literature cited in the description

- **J.BOEHLER.** *Nephrology Dialysis Transplantation,* 1993, vol. 8, 1359-1365
- **HIGUCHI.** *The Japanese Journal of Artificial Organs,* 1997, vol. 26 (4), 835-839
- **S.S.SCHMIDTMANN.** *Nephrology Dialysis Transplantation,* 1990, vol. 5, 600-603
- Amino Acid Fermentation. KYORITSU SHUPPAN CO., LTD, 1972, vol. 2, 43-45